# EUROPEAN PATENT SPECIFICATION

(11) **EP 1 475 274 B1**
(45) Date of publication and mention of the grant of the patent: **31.08.2011**
(21) Application number: 03252812.7
(22) Date of filing: 06.05.2003
(51) Int. Cl.: B60R 21/01, B60N 2/00

(54) **Seat occupant monitoring system and method**
System und Verfahren zum Überwachen einer Sitzbelegung
Dispositif et méthode de surveillance du passager d'un siège

(43) Date of publication of application: 10.11.2004
(73) Proprietor: Mitsubishi Electric Information Technology Centre Europe B.V., Guildford, Surrey GU2 5YD (GB); MITSUBISHI DENKI KABUSHIKI KAISHA, Chiyoda-ku Tokyo 100-8310 (JP)
(72) Inventor: Bober, Miroslaw, Merrow, Guildford, Surrey GU1 2SE (GB); Zaharia, Ramona, Guildford GU1 4AY (GB)
(74) Representative: Whitlock, Holly Elizabeth Ann

(56) References cited:
- DE-A- 4 212 018
- DE-A- 19 806 535
- US-A1- 2002 059 022
- US-B1- 6 392 550
- US-B1- 6 529 809

## Description

This invention relates to the measurement of a force distribution on a car seat caused by an occupant of the seat and, possibly, based on the measurement, making alterations to the occupant's environment, according to claims 1 and 11.

Many cars are produced with air bags which are designed to be deployed in the event of accident to prevent or lessen the injury to a car seat occupant. However, it has been found that the rate of deployment of the air bag which is optimal for an occupant of certain weight and size may not suit an occupant of a different weight and size. In particular, it has been found that the optimal rate for the deployment of an air bag for an adult occupant may result in unnecessary injury to a child. Furthermore, if an infant occupies the seat, it may be desirable to prevent deployment of the air bag completely.

It is known that by making one or more measurements of the pressure exerted by an occupant on the car seat, the characteristics of the occupant may be ascertained and the rate at which the air bag is deployed can be varied in accordance.

Pressure measurements may also be used to ascertain characteristics other than the weight and size of the occupant such as the location and position of the occupant in the seat. It is also known to use pressure measurements to determine whether the occupant's posture is correct and if not, to indicate a more preferable posture to the occupant of the car seat.

Such measurements are also used to control a seat belt pre-tensioner or to determine a driver alertness level.

US-B1-6 250 671 describes an occupant sensor and airbag control system. The disclosed system uses strain gauges employing resistors which alter their resistance according to the amount of force exerted on the gauge. The force on the car seat is measured at four points and these measurements are used to calculate the weight and centre of gravity of a car seat occupant. The centre of gravity and the weight are combined with a measurement of an inclination of the car sear to determine the rate at which an air bag should be deployed.

US-A-5 573 269 also discloses a system whereby the force exerted by a seat occupant is measured and combined with a measurement of seat inclination to determine the rate at which an air bag is to be deployed. Strain gauges measure the deformation of a plate disposed below or in a seat cushion caused by an occupant of the seat and the force is calculated from these measurements.

US-B1-6 246 936 discloses apparatus which distinguishes between a child or small adult occupant of a car seat and a child seat placed on the car seat by measuring the weight variance of the occupant. The total pressure of a liquid in a bladder disposed in the car seat is measured and this measurement is used to determine the weight of the occupant. Variations in the pressure during movement of the vehicle as well as the weight are used to determine whether the occupant is an infant in a child seat or an adult. The deployment of an air bag is varied accordingly.

US-B1-6 348 663 discloses a method for determining parameters of a seat occupant which involves subdividing the seat into at least two sections and measuring the centre of mass of each section. The centre of mass of each section is determined from measurements of force sensitive resistors.

US-B1-6 392 550, which is considered the closest prior art, discloses a method of determining driver alertness based on the force distribution which the driver exerts on a car seat. The force distribution is calculated from measurements of an array of variable resistors which alter their resistance in dependence on the pressure exerted thereon. Driver alertness is ascertained by a neural network processing technique.

US-A1-2002/0059022 discusses a system for determining the occupancy state of a vehicle seat, and controlling a component of the vehicle in dependence on the determined occupancy state.

DE 198 06 535 A1 discusses an adaptive seating comfort control which automatically ajusts the pressure in at least one fluid filled bladder positioned in a seat in accordance with the sensed position of a seat occupant.

The aforementioned disclosures all use a sensor to measure the pressure exerted on a sensor. Sensors of the prior art which are capable of measuring a force distribution applied to a seat are reactive to the force applied to the seat near the sensor and to increase the area over which measurements can be made, more sensors are added. More sensors are also added to increase the number of measurements made. In practice however, the number of sensors which may be disposed is limited by the physical size of the sensors as well as their cost.

It is desirable to provide a reliable method and apparatus for estimating various parameters of a seat occupant such as the occupant's weight, build, size, location and orientation and, on the basis of this, to provide a method and apparatus for controlling a seat belt pre-tensioner, deploying an air bag and/or monitoring the level of comfort of seat adjustment for a particular occupant. The comfort level may be used to provide a method and apparatus for monitoring a level of driver alertness and, if such level falls below a predetermined threshold, for alerting the driver.

Aspects of the invention are set out in the accompanying claims.

According to a further aspect of the invention a sensor is provided for measuring a force distribution which includes transducer means reactive to the force distribution and means for making a plurality of measurements of the transducer means, each measurement being a respective different function of the force distribution. The sensor provides measurements which are each affected by force applied substantially anywhere to the sensor. This provides a high degree of sensitivity to changes of the force and the distribution of the force without employing a large number of discrete sensors.

The sensor may include a pad manufactured from a solid state material which changes its electrical properties when a force is applied to the material. The resistance or the capacitance of the material may change and the material may be a quantum tunnelling composite. Preferably, the pad is resistant to cracking, soft and it substantially preserves its mechanical properties over a 10 to 15 year period. The thermal behaviour of the pad is known, at least, in the range of -40°C to 60°C.

According to a further aspect of the invention an occupant monitoring apparatus includes at least one such sensor. The occupant may be an occupant of a seat and is preferably the occupant of a vehicle (e.g. car) seat. The occupant may be a person or any object such as luggage or a child seat.

The measurements may be processed using a neural network technique or other suitable pattern recognition technique such as support vector machines (SVM's).

The processing may classify the occupant according to dimensions, weight, location, position or orientation.

The system may alter at least one parameter of the occupant's environment according to the classification. Such parameters may include but are not limited to a rate at which an air bag is deployed, the operation of a seat belt pre-tensioner, a seat orientation or the display of information.

The preferred embodiment of the present invention contrasts with arrangements incorporating prior art sensors which were deliberately arranged so as to detect pressure confined, or substantially confined, to specified local areas, and which used rule-based criteria to generate an output dependent on the location of the sensors and the pressures sensed thereby.

In the preferred embodiment however, each sensor output is dependent on pressure over a large area, with multiple outputs representing pressure over a common area (albeit using different transfer functions). Rather than using a rule-based system for interpreting the outputs, a training-based classification technique is used.

Arrangements embodying the invention will now be described by way of example with reference to the accompanying drawings, in which:
Figure 1 illustrates a first embodiment of a sensor;
Figure 2 illustrates an alternative embodiment of a sensor;
Figure 3 illustrates a car seat incorporating a sensor of the type illustrated in Figures 1 and 2;
Figure 4 is a schematic representation of signal processing apparatus for processing signals generated by the sensors of Figures 1, 2 or 3.

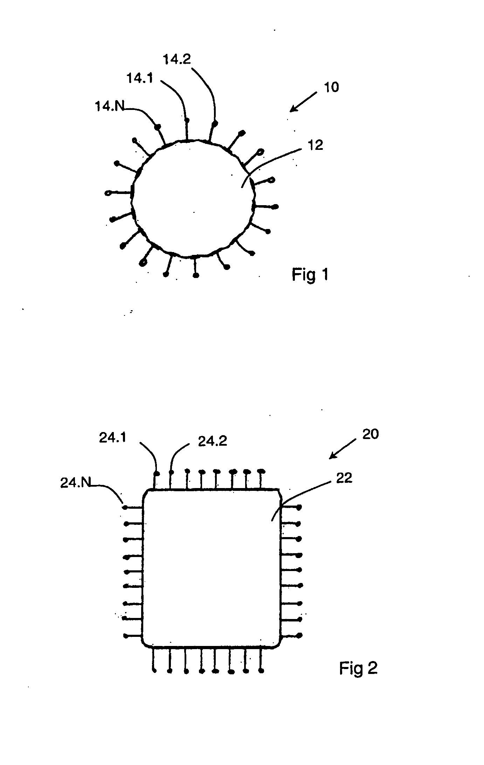

Referring to Figure 1, a sensor 10 includes a circular pad 12 and a plurality of electrodes 14.1, 14.2,... 14.N connected to the pad 12.

Figure 2 illustrates an alternative embodiment of the sensor of Figure 1. A sensor 20 includes a square pad 22 with a plurality of electrodes 24.1, 24.2, ... 24.N connected to the pad 22.

The resistive properties of each of the pads 12 and 22 illustrated in Figures 1 and 2 change depending on the force exerted on the pads. The pad may be manufactured from a quantum tunnelling composite (QTC) which has been treated to provide the appropriate shape. Alternatively, the pad may be manufactured from any of a variety of pressure sensitive materials of types well known in the art, which material preferably has isotropic electrical properties. Alternatively, other parameters of the pad could be measured such as capacitance.

Metal contacts are attached to the pad to form electrodes (14.1, 14.2, ... 14.N and 24.1, 24.2,... 24.N). A measurement of the current flowing between any two electrodes will be a respective function of the force distribution on the pad. By measuring the current flowing between respective pairs of electrodes a large number of measurements which are respective functions of the total force distribution applied to the pad can be collected.

The construction of the pads is such that the number of electrodes may be altered to suit the number of measurements which are required. The sensors 10 and 20 allow substantially more measurements of a force distribution over a given area than the sensors of the prior art.

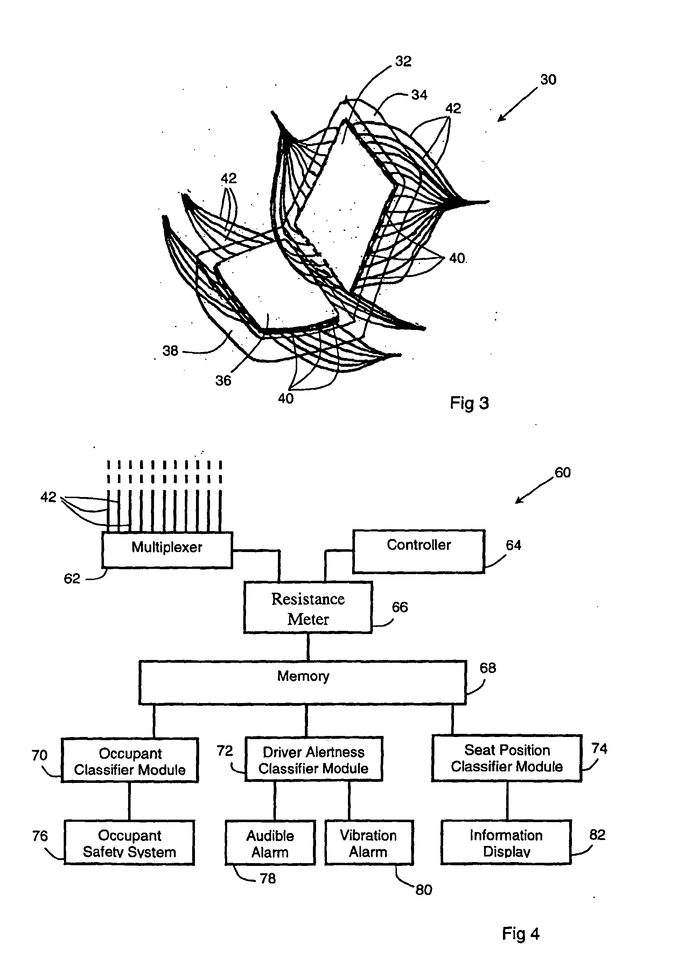

Figure 3 illustrates a car seat 30 which includes a pad 32 attached to a back rest 34 of the seat. A further pad 36 is attached to a seat bottom 38. Each of the pads 32 and 36 are of the same type as the pads 12 and 22 illustrated in Figures 1 and 2 and each includes a plurality of electrodes 40 which have connectors 42.

Figure 4 is a schematic illustration of a signal processing apparatus 60 which includes a multiplexer 62 to which the connectors 42 from the electrodes 40 illustrated in Figure 3 are connected. The multiplexer 62 is connected to a controller 64 which is in turn connected to a resistance meter 66 and a memory 68. The controller reads the signals generated by the electrodes 40 and converts this to a resistance measurement using the resistance meter 66. The resistance together with an identifier of the specific electrodes which produced that measurement are a representation of the force distribution over the pad and these measurements are stored in the memory 68. As the signals from each of the connectors 42 are multiplexed, only a single resistance meter 66 is required. The manner in which such measurements are made and stored is well known in the art of signal processing and will not be further described herein.

An occupant classifier module 70, a driver alertness classifier module 72 and a seat position classifier module 74 are all connected to the memory 68. The occupant classifier module 70 is connected to an occupant safety system 76. The driver alertness module 72 is connected to an audible alarm 78 and to a vibration alarm 80. The seat position classifier module 74 is connected to an information display 82.

The modules 70, 72 and 74 operate in a similar manner in that they all access the information stored in the memory 68 and use this information to classify a group of measurements. These controllers use support vector machines (SVM's) to classify the measurements. It is also possible to use a neural network based technique for each of these modules.

The classification is based on training each module to recognise a set of measurements as representative of a certain state. During a learning stage each module is programmed to associate a given set of measurements with a particular object and object orientation and position. These associations are then used by the modules during an operational stage to classify unknown measurements.

The use of SVM's as a tool to assist in classification problems is known in the field of physics. This is disclosed in "Support Vector Machines for Classification Problems in High Energy Physics" by M. Feindt and C. Haag, DELPHI Collaboration, DELPHI 99-151 GEN 176, 23 September, 199. SVM techniques are described in "An Introduction to Support Vector Machines and Other Kernel-based Learning Methods" by Nello Cristianini and John Shawe-Taylor (Cambridge University Press, 23 March, 2000) and in "Learning and Soft Computing" by Vojislav Kecman (The MIT Press, 24 July, 2001). Such techniques are well known in the art of measurement interpretation and will not be further described herein.

The occupant classifier module uses the technique to classify the occupant of the car seat. This module distinguishes between a child, an adult and an infant in a child seat occupying the seat. The module is also capable of distinguishing an object such as a piece of baggage occupying the seat from a human occupant.

The occupant classifier module indicates the class of the occupant to the occupant safety system 76 which determines whether an air bag is deployed and, if so, varies the rate of deployment. The occupant safety system 76 also adjusts a seat belt pre-tensioner according to the class of the occupant. This system may also control other safety features which can be optimised according to the determined class of the occupier.

The driver alertness classifier module distinguishes an alert driver from a drowsy or distracted driver. If this module determines that the driver is not alert, a vibration alarm 80 will be actuated to restore the driver to alertness. Alternatively or in addition to the vibration alarm, an audible alarm 78 may sound in slowly increasing loudness to alert the driver. In an alternative embodiment, the driver alertness classification module additionally receives information from the car steering (not illustrated) to indicate whether the driver is weaving which may also be indicative of the driver's alertness.

The seat position classifier module 82 determines whether the seat position for an occupant is the most comfortable possible. This module displays information to the seat occupant by means of the information display 82 indicating in what manner the orientation of the seat may be altered to increase comfort. To reduce the risk of accident, this module will only display the information when the car is stationary. In the case of a driver of the car, there may be a conflict between the driver alertness module and the seat position classifier module and it is therefore necessary to include a means of arbitration (not shown) between these modules which suppresses the seat position classifier module to ensure that the driver remains alert.

Although the controller is able to generate a measurement corresponding to each possible pair of electrodes, this may not be necessary. Depending on the number of electrodes which are included with the pads 32 and 36, the controller may reduce the number of measurements taken. The controller will therefore, based on the particular force distribution, omit measurements between those pairs of electrodes which would have no or little relevance to the characterisation of the force distribution. This decision may also be based on neural network or SVM techniques.

The sensor of the type illustrated and discussed with reference to Figures 1, 2 and 3 has many more applications than that of monitoring a car seat occupant. Such sensors may find a use in any application where a force distribution over a particular area is required to be known in detail.

It is to be realised that it is not necessary for the purposes of the current invention to directly correlate the measurements to the force being applied or the actual locations of the electrodes because of the use of training based classifiers.

## Claims

1. A method of monitoring a seat occupant which includes the steps of:
measuring a force distribution applied on the seat (30) by the seat occupant using at least one sensor, and
classifying the seat occupant on the basis of said force distribution,
**characterised in that**:
said measuring step comprises, for a sensor (10,20) including a plurality of pairs of electrodes (14,24) connected to a sensor pad (12,22), making a plurality of measurements with each measurement representing an electrical property measured between a respective pair of electrodes (14,24) and being dependent on the force applied over substantially all of the sensor pad (12,22) in accordance with a respective different function of the force distribution; and
said classifying step comprises classifying the seat occupant on the basis of said plurality of measurements from said sensor (10,20) representative of said force distribution.

2. The method according to claim 1 wherein each measurement represents a resistance measured between a respective pair of electrodes.

3. The method according to any preceding claim further comprising the step of storing each measurement together with a measurement identifier representing the respective measurement function.

4. The method according to any preceding claim wherein the step of classifying the seat occupant includes the step of using a trained classifier.

5. The method according to claim 4 wherein the classifier has been trained using a neural network.

6. The method according to claim 4 wherein the classifier has been trained using a support vector machine.

7. The method according to any preceding claim wherein said classifying step classifies a type of occupant.

8. The method according to any preceding claim wherein said classifying step classifies a location or orientation of the occupant.

9. The method according to claim 7 or 8 which includes the step of altering the occupant's environment according to the classification of the occupant.

10. The method according to claim 10 wherein the seat is a vehicle seat and which includes the step of varying any one or more of air bag deployment, seat belt pre-tensioner or information presentation according to the classification of the occupant.

11. Apparatus for monitoring a seat occupant, **characterised by**:
at least one sensor (10,20) which includes a sensor pad (12,22) and a plurality of pairs of electrodes (14,24) attached to the sensor pad (12,22);
measurement means (60) attached to the electrodes for making a plurality of measurements, wherein each measurement represents; an electrical property measured between a respective pair of electrodes and is dependent on the force applied over substantially all of the sensor pad in accordance with a respective different function of the force distribution over the sensor pad; and
classifying means (70) tor classifying the seat occupant on the basis of the plurality of measurements.

## Patentansprüche

1. Verfahren zum Überwachen eines Insassen auf einem Sitz, umfassend folgende Schritte:
Messen einer Kraftverteilung, die von dem Insassen auf den Sitz (30) ausgeübt wird, unter Verwendung mindestens eines Sensors, und
Klassifizieren des Insassen auf der Grundlage der Kraftverteilung,
**dadurch gekennzeichnet, dass**:
der Schritt des Messens für einen Sensor (10, 20), der eine Vielzahl von Elektrodenpaaren (14, 24) umfasst, die an ein Sensorfeld (12, 22) angeschlossen sind, das vornehmen einer Vielzahl von Messungen umfasst, wobei jede Messung eine elektrische Eigenschaft darstellt, die zwischen einem jeweiligen Elektrodenpaar (14, 24) gemessen wird und von der Kraft abhängig ist, die im Wesentlichen über das ganze Sensorfeld (12, 22) gemäß einer jeweiligen anderen Funktion der Kraftverteilung ausgeübt wird; und
der Schritt des Klassifizierens das Klassifizieren des Insassen auf der Grundlage der Vielzahl von Messungen von dem Sensor (10, 20), die für die Kraftverteilung repräsentativ sind, umfasst.

2. Verfahren nach Anspruch 1, wobei jede Messung einen Widerstand darstellt, der zwischen einem jeweiligen Elektrodenpaar gemessen wird.

3. Verfahren nach einem der vorhergehenden Ansprüche, ferner umfassend den Schritt des Speicherns jeder Messung zusammen mit einem Messungsidentifizierer, der die jeweilige Messfunktion darstellt.

4. Verfahren nach einem der vorhergehenden Ansprüche, wobei der Schritt des Klassifizierens des Insassen den Schritt des Verwendens eines geschulten Klassifikators umfasst.

5. Verfahren nach Anspruch 4, wobei der Klassifikator unter Verwendung eines neuronalen Netzes geschult wurde.

6. Verfahren nach Anspruch 4, wobei der Klassifikator unter Verwendung einer Trägervektormaschine geschult wurde.

7. Verfahren nach einem der vorhergehenden Ansprüche, wobei der Schritt des Klassifizierens eine Insassenart klassifiziert.

8. verfahren nach einem der vorhergehenden Ansprüche, wobei der Schritt des Klassifizierens eine Position oder Orientierung des Insassen klassifiziert.

9. Verfahren nach Anspruch 7 oder 8, umfassend den Schritt des Änderns der Umgebung des Insassen gemäß der Klassifizierung des Insassen.

10. Verfahren nach Anspruch 10, wobei der Sitz ein Fahrzeugsitz ist, und umfassend den Schritt des Variierens eines oder mehrerer Elemente von Luftsackentfaltung, Sitzgurtstraffer oder Informationsanzeige gemäß der Klassifizierung des Insassen umfasst.

11. Gerät zum Überwachen eines Insassen auf einem Sitz, **gekennzeichnet durch**:
mindestens einen Sensor (10, 20), der ein Sensorfeld (12, 22) und eine Vielzahl von Elektrodenpaaren (14, 24), die an dem Sensorfeld (12, 22) angebracht sind, umfasst;
Messmittel (60), die an den Elektroden angebracht sind, um eine Vielzahl von Messungen vorzunehmen, wobei jede Messung eine elektrische Eigenschaft darstellt, die zwischen einem jeweiligen Elektrodenpaar gemessen wird und von der Kraft abhängig ist, die gemäß einer jeweiligen anderen Funktion der Kraftverteilung über das Sensorfeld im Wesentlichen auf das ganze Sensorfeld ausgeübt wird; und
Klassifizierungsmittel (70) zum Klassifizieren des Insassen auf der Grundlage der Vielzahl von Messungen.

## Revendications

1. Procédé de surveillance d'un occupant de siège qui comprend les étapes consistant à:
mesurer une répartition de force appliquée sur le siège (30) par l'occupant du siège au moyen d'au moins un capteur, et
classifier l'occupant du siège sur la base de ladite répartition de force,
**caractérisé en ce que**
ladite étape de mesure consiste, pour un capteur (10, 20) comprenant une pluralité de paires d'électrodes (14, 24) connectées à une pastille de capteur (12, 22), à réaliser une pluralité de mesures, chaque mesure représentant une propriété électrique mesurée entre une paire respective d'électrodes (14, 24) et étant dépendante de la force appliquée sur sensiblement la totalité de la pastille de capteur (12, 22) selon une fonction différente respective de la répartition de force; et
ladite étape de classification consiste à classifier l'occupant du siège sur la base de ladite pluralité de mesures provenant dudit capteur (10, 20) représentatives de ladite répartition de force.

2. Procédé selon la revendication 1, dans lequel chaque mesure représente une résistance mesurée entre une paire respective d'électrodes.

3. Procécé selon l'une quelconque des revendications précédentes comprenant en outre l'étape de stockage de chaque mesure conjointement avec un identifiant de mesure représentant la fonction de mesure respective.

4. Procédé selon l'une quelconque des revendications précédentes dans lequel l'étape de classification de l'occupant du siège comprend l'étape de l'utilisation d'un classificateur entraîné.

5. Procédé selon la revendication 4, dans lequel le classificateur a été entraîné au moyen d'un réseau neuronal.

6. Procédé selon la revendication 4, dans lequel le classificateur a été entraîné au moyen d'une machine à vecteurs de support.

7. Procédé selon l'une quelconque des revendications précédentes dans lequel ladite étape de classification classifie un type d'occupant.

8. Procédé selon l'une quelconque des revendications précédentes, dans lequel ladite étape de classification classifie un emplacement ou une orientation de l'occupant.

9. Procédé selon la revendication 7 ou 8 qui comprend l'étape de modification de l'environnement de l'occupant selon la classification de l'occupant.

10. Procédé selon la revendication 10, dans lequel le siège est un siège de véhicule et qui comprend l'étape de variation d'un ou plusieurs éléments parmi le déploiement d'un coussin de sécurité gonflable, le prétendeur de ceinture de sécurité ou la présentation d'informations selon la classification de l'occupant.

11. Appareil de surveillance d'un occupant de siège, **caractérisé par** :
au moins un capteur (10, 20) qui comprend une pastille de capteur (12, 22) et une pluralité de paires d'électrodes (14, 24) fixées à la pastille de capteur (12, 22);
des moyens de mesure (60) fixés aux électrodes pour réaliser une pluralité de mesures, dans lesquels chaque mesure représente une propriété électrique mesurée entre une paire respective d'électrodes et dépend de la force appliquée sur sensiblement la totalité de la pastille du capteur selon une fonction différente respective de la répartition de force sur la pastille du capteur ; et
des moyens de classification (70) pour classifier l'occupant du siège sur la base de la pluralité de mesures.
